# FASCICULE DE BREVET EUROPEEN

(11) **EP 3 119 813 B1**
(45) Date de publication et mention de la délivrance du brevet: **04.01.2023**
(21) Numéro de dépôt: 15717196.8
(22) Date de dépôt: 20.03.2015
(51) Int. Cl.: C08B 30/04, C08B 30/18, G01N 33/50, G01N 33/68

(54) **PROCEDE OPTIMISE DE DECONTAMINATION DE PRODUCTION DE POLYMERES DE GLUCOSE ET D'HYDROLYSATS DE POLYMERES DE GLUCOSE**
OPTIMIERTES VERFAHREN ZUR DEKONTAMINATION DER HERSTELLUNG VON GLUCOSEPOLYMEREN UND GLUCOSEHYDROLYSATEN
OPTIMIZED METHOD FOR DECONTAMINATING PRODUCTION OF GLUCOSE POLYMERS AND GLUCOSE POLYMER HYDROLYSATES

(30) Priorité: 21.03.2014 FR 1452354
(43) Date de publication de la demande: 25.01.2017
(73) Titulaire: Roquette Frères, 62136 Lestrem (FR)
(72) Inventeur: LANOS, Pierre, F-59480 La Bassee (FR); DUVET, Sophie, F-59890 Quesnoy Sur Deule (FR); DUPONT, Thierry, F-62232 Vendin Les Bethune (FR); ALLAIN, Fabrice, F-59800 Lille (FR); CARPENTIER, Mathieu, F-59350 Saint Andre Lez Lille (FR); DENYS, Agnès, F-59800 Lille (FR); HACINE-GHERBI, Héla, F-59650 Villeneuve D'ascq (FR)
(74) Mandataire: Plasseraud IP
(86) Numéro de dépôt international: PCT/FR2015/050706
(87) Numéro de publication internationale: WO 2015/140477

(56) Documents cités:
- WO-A1-2012/059685
- WO-A1-2012/143647
- WO-A1-2013/178931

## Description

La présente invention est relative à l'élaboration d'un procédé optimisé de décontamination des circuits de production ou de purification de polymères de glucose, plus particulièrement ceux destinés aux domaines alimentaires (ingrédients santés riches en fibres) et médicaux (dialyse péritonéale), ou d'hydrolysats de polymères de glucose, plus particulièrement ceux destinés aux domaines médicaux (glucose apyrogène injectable).

### Arrière-plan technologique de l'invention

La société Demanderesse a choisi de développer son invention dans un domaine connu pour la dangerosité des contaminants d'origine microbienne susceptibles de se développer dans les circuits de production des polymères de glucose ou dans ceux de production de leurs hydrolysats, contaminants à l'origine de possibles :
- intoxications alimentaires,
- réactions inflammatoires très néfastes pour la santé humaine.

Dans le cadre d'une démarche de sécurité alimentaire, comme dans celui d'une démarche de sécurité sanitaire, il est donc important de s'assurer de l'absence de contaminants d'origine microbienne, tant sous forme de cellules vivantes que de débris cellulaires, par tous les moyens techniques adéquats, notamment :
- la définition de méthodes d'identification et de dosage efficaces des contaminants,
- la définition de circuits de production sécurisés, par la mise en place de dispositifs et de techniques de purification adaptées.

Dans le cas de la dialyse péritonéale, un certain nombre d'ingrédients doivent être préparés dans les conditions de pureté les plus strictes.

La dialyse péritonéale est en effet un type de dialyse qui a pour objectif d'éliminer les déchets tels que l'urée, la créatinine, l'excès de potassium ou l'excédent d'eau que les reins ne parviennent pas ou plus à épurer du plasma sanguin. Ce traitement médical est indiqué en cas d'insuffisance rénale chronique terminale.

Les dialysats les plus couramment utilisés sont composés d'une solution tampon (du lactate ou du bicarbonate) à pH acide (5,2 - 5,5) ou physiologique (7,4) à laquelle sont ajoutés :
- des électrolytes (sodium, calcium, magnésium, chlore) et surtout
- un agent osmotique, principalement un polymère de glucose, tel que par exemple l'« icodextrine » présent dans la solution pour dialyse péritonéale ambulatoire EXTRANEAL^{®} commercialisée par la société BAXTER.

Dans ce domaine plus particulier de l'utilisation des polymères du glucose destinés à la dialyse péritonéale continue et ambulatoire, il est très vite apparu que ces hydrolysats d'amidon (mélange de glucose, d'oligomères et de polymères de glucose) ne pouvaient pas être utilisés tels quels.

La demande de brevet européen EP 207.676 enseigne qu'il est préféré des polymères de glucose formant des solutions limpides et incolores à 10 % dans l'eau, ayant un poids moléculaire moyen en poids (Mw) de 5.000 à 100.000 daltons et un poids moléculaire moyen en nombre (Mn) inférieur à 8.000 daltons.

De tels polymères de glucose comprennent aussi de façon préférée au moins 80 % de polymères du glucose dont le poids moléculaire est compris entre 5.000 et 50.000 daltons, peu ou pas de glucose ou de polymères du glucose de DP inférieur ou égal à 3 (poids moléculaire 504) et peu ou pas de polymères de glucose de poids moléculaire supérieur à 100.000 (DP voisin de 600).

En d'autres termes, les polymères de glucose préférés sont des polymères de glucose de faible indice de polymolécularité (valeur obtenue en calculant le rapport Mw/Mn).

Les procédés proposés dans cette demande de brevet EP 207.676 pour obtenir ces polymères de glucose de faible indice de polymolécularité à partir d'hydrolysats d'amidon consistent :
- soit à effectuer une précipitation fractionnée d'une maltodextrine à l'aide d'un solvant miscible à l'eau,
- soit à effectuer une filtration moléculaire de cette même maltodextrine au travers de différentes membranes possédant un seuil de coupure ou d'exclusion adéquat.

Dans les deux cas, ces procédés visent à éliminer à la fois les polymères de très haut poids moléculaire et les monomères ou oligomères de faible poids moléculaire.

Ces procédés ne donnent toutefois pas satisfaction tant du point de vue de leur mise en oeuvre que du point de vue des rendements et de la qualité des produits qu'ils permettent d'obtenir.

Soucieuse de mettre au point un procédé de fabrication d'un polymère de glucose complètement soluble dans l'eau et de faible indice de polymolécularité préférentiellement inférieur à 2,5, ayant de préférence un Mn inférieur à 8.000 daltons et possédant un Mw compris entre 12.000 et 20.000 daltons, procédé qui soit dépourvu des inconvénients de l'art antérieur, la société Demanderesse, s'est attachée à résoudre ce problème dans son brevet EP 667.356, en partant d'un amidon hydrolysé, plutôt que d'une maltodextrine.

Le polymère de glucose obtenu par fractionnement chromatographique contient alors de préférence moins de 3 % de glucose et de polymères de glucose de DP inférieur ou égal à 3 et moins de 0,5 % de polymères de glucose de DP supérieur à 600.

Il est finalement désormais admis par les experts du domaine de la dialyse péritonéale que ces polymères de glucose, utilisés pour leur pouvoir osmotique, donnent toute satisfaction.

### Les risques de contamination

Il est cependant à déplorer des risques de contamination microbienne des préparations destinées à la dialyse péritonéale.

Il est en effet connu que les circuits de production des polymères de glucose peuvent être contaminés par des microorganismes, ou par des substances pro-inflammatoires contenus dans lesdits microorganismes.

Il est par exemple décrit en amidonnerie la contamination des amidons de maïs ou de blé par des microorganismes de type levures, moisissures et bactéries, et plus particulièrement par des bactéries acidothermophiles de type *Alicyclobacillus acidocaldarius* (bactéries extrémophiles qui se développent dans les zones chaudes et acides du circuit).

Le risque majeur pour le patient qui reçoit ces produits contaminés est alors la péritonite.

Ces épisodes de péritonite sont provoqués par des infections bactériennes intrapéritonéales, et le diagnostic est habituellement facilement établi par les cultures positives de dialysat.

La « péritonite stérile », décrite en tant que péritonite aseptique, chimique, ou culture-négative, est quant à elle typiquement provoquée par un irritant chimique ou un corps étranger.

Depuis l'introduction de l'icodextrine pour la préparation de solutions de dialyse péritonéale, des cas isolés de péritonite aseptique ont été rapportés, pouvant être liés à des causes diverses et notamment l'induction par des substances pro-inflammatoires potentiellement présentes.

Les épisodes inflammatoires aseptiques sont donc des complications majeures observées après injections de solutions de dialyse.

Si une partie de ces épisodes inflammatoires est liée à un problème d'ordre chimique (injection accidentelle de contaminants chimiques ou mauvais dosages de certains composés), la majorité des cas est directement associée à la présence de contaminants d'origine microbienne présents dans les solutions servant à la préparation des solutions de dialyse.

Les lipopolysaccharides (LPS) et les peptidoglycanes (PGN) sont les principaux contaminants d'origine microbienne présentant un risque élevé de déclencher une inflammation même lorsqu'ils sont présents à l'état de traces.

Il est par ailleurs du mérite de la société Demanderesse d'avoir également pris en compte la présence de molécules susceptibles d'exacerber la réponse inflammatoire induite par ces contaminants, tels que les produits de dépolymérisation des PGN, dont la structure minimale encore bioactive est le muramyl-dipeptide (MDP).

En plus des produits de dépolymérisation des PGN, des peptides microbiens formylés, dont le prototype est le f-MLP (tripeptide formyl-Met-Leu-Phe), ont également une activité synergique importante. A l'origine, ces peptides ont été identifiés pour leur activité chimio-attractante sur les leucocytes, alors qu'ils sont incapables d'induire une réponse cytokinique *per se.*

Il est donc important de ne pas négliger ces "petites molécules", car elles peuvent rendre compte indirectement des épisodes inflammatoires aseptiques en exacerbant les effets de traces de PGN et/ou de LPS.

### Définition de méthodes d'identification et de dosage efficaces desdits contaminants

La société Demanderesse s'est donc employée à développer des méthodes de détection et de dosage plus efficaces que ceux accessibles dans l'état de l'art.

Ces dernières années, de nombreux tests utilisant des cellules primaires se sont développés pour remplacer les modèles animaux dans les tests de réponse inflammatoire.

Toutefois, ces modèles *in vitro* sont sujets à une importante variabilité interindividuelle, ce qui peut être responsable de biais expérimentaux.

A l'inverse, les lignées cellulaires monocytaires donnent des réponses constantes, ce qui explique pourquoi les tests actuellement en développement utilisent de plus en plus ce type de cellules en culture. Cependant, ces tests présentent l'inconvénient de donner une réponse inflammatoire globale à tous les contaminants présents en mélange dans une solution, et par conséquent ne permettent pas de caractériser la nature du contaminant.

Il est également important de noter que la réponse inflammatoire exacerbée est visible pour les cytokines de la phase aiguë de l'inflammation, telles que :
- TNF-α (Tumor Necrosis Factor alpha),
- IL-1β (interleukine 1β) et
- les chimiokines telles que CCL5 (Chemokine (C-C motif) ligand 5) /RANTES (Regulated upon Activation, Normal T-cell Expressed, and Secreted), mais pas ou peu pour l'IL-6 (interleukine 6).

Ainsi, les méthodes basées sur la production de l'IL-6 (US 2009/0239819 et US 2007/0184496) ne sont pas adaptées pour détecter des contaminants en mélange dans une solution.

Il a donc été du mérite de la société Demanderesse d'avoir développé dans sa demande de brevet internationale WO 2012/143647 des méthodes sensibles et efficaces de détection des contaminants microbiens ayant une action pro-inflammatoire, en deçà du seuil de sensibilité des procédures actuellement utilisées et/ou décrites dans la littérature, et ultérieurement d'avoir identifié la famille, voire la nature, des molécules pro-inflammatoires présentes sous formes de traces dans les lots provenant des circuits de production.

### Détermination de l'efficacité des étapes unitaires de purification

La société Demanderesse a ensuite cherché à mieux définir les étapes clefs de purification à mettre en oeuvre pour assurer une sécurité optimale des lignes de production, notamment des polymères de glucose.

Pour cela, elle s'est employée à valider les étapes clefs unitaires de purification desdits circuits, en employant les méthodes de détection et dosages basées sur les lignées monocytaires telles que présentées dans sa demande de brevet internationale WO 2012/143647.

Ainsi, dans sa demande de brevet internationale WO 2013/178931, la société Demanderesse a analysé l'efficacité des étapes unitaires suivantes :
- traitement thermique,
- acidification,
- passage sur charbon actif,
- passage sur des résines d'adsorption, d'ultrafiltration, de filtration,
- hydrolyse chimique ou enzymatique.

Pour analyser l'efficacité de ces différentes étapes unitaires, différentes étapes de décontamination ont alors été réalisées sur différentes matrices :
- des polymères de glucose, matières premières de l'Icodextrine (avant fractionnement chromatographique selon l'enseignement du brevet EP 667.356),
- un lot d'icodextrine,
- un lot de maltodextrine branchée, commercialisé par la société Demanderesse sous le nom de marque NUTRIOSE^{®} FB06,
- un lot de dextrose monohydrate préparé pour être conditionné en solution injectable, commercialisé par la société Demanderesse sous le nom de marque LYCADEX^{®} PF,
- un lot de polymères solubles de glucose hautement branchés préparé selon l'enseignement de la demande de brevet internationale WO 2007/099212 dont la société Demanderesse est titulaire,
- une maltodextrine commerciale.

Si ces différents travaux ont permis de démontrer l'incidence que pourrait avoir chacune de ces étapes sur l'élimination de contaminants donnés pour chacune des matrices, il fallait encore définir la meilleure combinaison apte à sécuriser toutes matrices de tous contaminants potentiels.

De l'ensemble des résultats présentés dans la demande de brevet WO 2013/178931, il demeurait donc encore un besoin non satisfait d'élaborer un procédé optimisé de décontamination des circuits de production ou de purification de polymères de glucose, plus particulièrement ceux destinés aux domaines alimentaires (ingrédients santés riches en fibres) et médicaux (dialyse péritonéale), ou d'hydrolysats de polymères de glucose, plus particulièrement ceux destinés aux domaines médicaux (glucose apyrogène injectable).

### Résumé de l'invention

La présente invention propose donc une combinaison de plusieurs étapes de décontamination judicieusement sélectionnées et ordonnées qui se révèle efficace pour éliminer toutes les molécules inflammatoires susceptibles d'être présentes dans les circuits de production, notamment des polymères de glucose sélectionnés parmi l'icodextrine et les maltodextrine, quelle que soit la nature de la contamination.

Le procédé de l'invention se rapporte ainsi à la combinaison d'étapes suivante dans l'ordre suivant :
- traitement par une préparation enzymatique à activité mannanase,
- traitement par un charbon actif de très haute capacité d'adsorption, de qualité pharmaceutique, et de porosité « micropore » ;
- facultativement, traitement par un second charbon actif de porosité « mésopore »,
- passage sur une résine adsorbante polymérique macroporeuse, présentant une porosité supérieure à 100 × 10⁻¹⁰ m ; et
- ultrafiltration en continu sur 5 kDa.

Au sens de l'invention, on entend par :
- « préparation enzymatique à activité mannanase », une préparation telle la Mannaway^{®} commercialisé par la société Novozymes ;
- « charbon actif de très haute capacité d'adsorption, de qualité pharmaceutique, et de porosité « micropore » : un charbon actif de porosité équivalente au charbon actif Norit C Extra USP ;
- « charbon actif de porosité « mésopore » : un charbon actif de porosité équivalente au charbon actif ENO-PC ;
- « résine adsorbante polymérique macroporeuse, présentant une porosité supérieure à 100 Angström » : une résine de type DOWEX OPTIDORE SD2.

De préférence, le procédé comprend les 5 étapes.

Les polymères de glucose sont sélectionnés parmi de l'icodextrine et des maltodextrines, en particulier des maltodextrines branchées ou non, et les hydrolysats de polymères de glucose sont un produit d'hydrolyse totale tel le dextrose monohydrate

Les étapes sélectionnées permettent de cibler les différentes familles de contaminants et ainsi d'obtenir des produits exempts de réactivité inflammatoire.

### Description détaillée de l'invention

Le procédé de l'invention est destiné à se substituer aux voies classiquement mises en œuvre pour purifier les polymères de glucose ou leurs hydrolysats.

Le procédé de décontamination des polymères de glucose ou leurs hydrolysats de leurs molécules pro-inflammatoires, conforme à l'invention, comprend les étapes suivantes :
a) fournir des polymères de glucose ou leurs hydrolysats ;
b) facultativement, détecter ou doser les molécules pro-inflammatoires dans les polymères de glucose ou leurs hydrolysats fournis à l'étape a) ;
c) effectuer les étapes de purification suivantes dans l'ordre suivant:
   i. traitement par une préparation enzymatique à activité mannanase,
   ii. traitement par un charbon actif de très haute capacité d'adsorption, de qualité pharmaceutique, de porosité « micropore »,
   iii. facultativement, traitement par un second charbon actif de porosité « mésopore » ,
   iv. passage sur une résine adsorbante polymérique macroporeuse, présentant une porosité supérieure à 100 × 10⁻¹⁰ m, et
   v. ultrafiltration en continu sur 5 kDa
dans lequel les polymères de glucose sont sélectionnés parmi l'icodextrine et les maltodextrines.

Les polymères de glucose ou leurs hydrolysats peuvent être destinés à la dialyse péritonéale, la nutrition entérale et parentérale, et l'alimentation des nouveaux nés.

Les polymères de glucose, qui seront préparés dans le cadre de la présente invention, sont de l'icodextrine ou des maltodextrines (branchées ou non, comme il sera décrit ci-après).

Les hydrolysats de polymères de glucose dont il est question ici s'entendent notamment du produit d'hydrolyse totale tel le dextrose monohydrate apyrogène, commercialisé sous la marque LYCADEX^{®} PF par la société Demanderesse.

Ils peuvent être décontaminés à un ou plusieurs stades de leur préparation, et notamment au niveau des dernières étapes de leur procédé de préparation.

Ainsi, les polymères de glucose ou leurs hydrolysats fournis dans les procédés selon la présente invention correspondent au produit précédant le produit final.

Les contaminants pro-inflammatoires sont surtout des molécules d'origine bactérienne.

Ils peuvent être en particulier :
- des PGN,
- des LPS,
- des lipopeptides,
- des produits de dépolymérisation des PGN, notamment du MDP,
- les peptides microbiens formylés comme le f-MLP,
- des β glucanes,
- etc...

Les méthodes de mesure des réponses inflammatoires *in vitro* qui sont utilisées dans le cadre de la présente invention pour suivre l'efficacité des étapes de décontamination des procédés de préparation de polymères de glucose à usage thérapeutique chez l'Homme (e.g. solutions de dialyse péritonéale) sont basées sur les tests cellulaires (« *bio-assays »*) utilisant des lignées de type monocytes/macrophages (THP-1, et/ou Raw-Blue^{™}) et des lignées transfectées exprimant un récepteur spécifique de l'immunité naturelle (HEK-Blue^{™}), tests cellulaires développés par la société Demanderesse et détaillés dans ses demandes de brevet antérieures.

Cinq lignées sont de préférence utilisées :
- Lignée Raw-Blue^{™} : cette lignée issue de macrophages murins répond à la majorité des contaminants pro-inflammatoires susceptibles d'être présents dans les matrices et dérivés de polymères de glucose (PGN, lipopeptides, LPS, zymosan, LTA). Son utilisation permet donc d'estimer la charge globale en molécules pro-inflammatoires présentes dans les échantillons.

- lignée HEK-Blue^{™} hTLR2 : cette lignée exprimant le récepteur hTLR2 répond spécifiquement aux agonistes du TLR2 (PGN et lipopeptides surtout). Son utilisation permet donc de connaître le taux de ces contaminants dans le déclenchement des réponses inflammatoires,
- lignée HEK-Blue^{™} hTLR4 : cette lignée exprimant le récepteur hTLR4 répond spécifiquement aux LPS. Son utilisation permet donc de connaître le taux de ces contaminants dans le déclenchement des réponses inflammatoires,
- lignée HEK-Blue^{™} hNOD2 : cette lignée exprimant le récepteur hNOD2 répond spécifiquement aux agonistes de NOD2. Son utilisation permet donc de connaître le taux de MDP et molécules apparentées dans le déclenchement des réponses inflammatoires,
- lignée HEK-Blue^{™} Null2: il s'agit d'une lignée contrôle, non transfectée par un récepteur de l'immunité. Son utilisation est nécessaire pour vérifier que les solutions de polymères de glucose ou de leurs hydrolysats n'induisent pas la production de la SEAP par un mécanisme de toxicité.

Cependant, il est à noter que l'homme du métier peut également utiliser d'autres lignées commerciales (IMGENEX) ou il peut en préparer.

Dans un mode de réalisation préféré, les lignées cellulaires sont mises en œuvre à une densité comprise entre 0,5 à 1 × 10⁶ cellules/mL de milieu de culture, et la mise en contact de la préparation de polymères de glucose ou leurs hydrolysats avec les cellules dure environ 16 à 24 h .

Une quantification des contaminants peut être réalisée à l'aide d'une courbe dose-réponse. Cette courbe dose-réponse peut notamment être réalisée avec les mêmes cellules, dans les mêmes conditions, avec des doses croissantes de contaminants. Les courbes doses-réponses sont en particulier réalisées avec des standards de LPS, PGN, lipopeptide et MDP.

De préférence, une telle courbe dose-réponse peut être réalisée pour les cellules exprimant TLR4 (par exemple, THP-1, HEK-Blue^{™} hTLR4 et Raw-Blue^{™}) avec des doses croissantes de LPS, pour les cellules exprimant TLR2 (par exemple, THP-1, HEK-Blue^{™} hTLR2 et Raw-Blue^{™}) avec des doses croissantes de PGN, et pour les cellules réactives via NOD2 (par exemple, HEK-Blue^{™} hNOD2) avec des doses croissantes de MDP.

Les tests cellulaires peuvent être réalisés comme décrit dans les demandes de brevet de la Demanderesse : WO2012/143647 et WO2013/178931.

La première étape de décontamination du procédé conforme à l'invention consiste en un traitement par une préparation enzymatique à activité mannanase, telle la préparation enzymatique Mannaway^{®} commercialisée par la société Novozymes qui s'est révélée efficace pour dissocier les macro-complexes tels que des débris bactériens et des PGN de poids moléculaire élevé.

Son activité est optimale lorsqu'elle est utilisée à une concentration finale de 0,4 % (vol/vol) dans la solution de polymères de glucose à 32 % (poids/vol) ajustée à pH 10 avec NaOH, pendant un temps de traitement de 24 h à 50°C.

Après traitement, la solution est neutralisée par HCI et l'enzyme est inactivée par chauffage à 85°C pendant 10 min.

La seconde étape consiste en un traitement par un charbon actif de très haute capacité d'adsorption, de qualité pharmaceutique, de porosité « micropore ».

La société Demanderesse recommande d'utiliser un charbon actif de type Norit C Extra USP. Le charbon C-extra-USP se montre en effet efficace pour éliminer les PGN et leurs produits de dégradation.

Son action est maximale lorsqu'il est ajouté à la concentration finale de 0,5% (poids/volume) dans la solution de polymères de glucose à 32 % (poids/vol), ajustée à pH 4,5 avec HCI. Le traitement est réalisé sous agitation pendant 1 h à 80°C. Après traitement, la solution est neutralisée par NaOH puis filtrée sur 0,22 µm.

La troisième étape consiste en un traitement par un second charbon actif de porosité « mésopore ». Cette étape est facultative.

Ici, un charbon actif de type ENO-PC est préféré. Cette qualité de charbon actif a un spectre d'action large et permet d'éliminer préférentiellement les molécules de poids moléculaire < 100 kDa (par exemple, LPS et produits de dégradation des PGN).

Il est également utilisé ici à une teneur de 0,5% à pH 4,5 pendant 1 h à une température de 80°C.

La quatrième étape consiste en un traitement sur une résine adsorbante polymérique macroporeuse, présentant une porosité supérieure à 100 × 10⁻¹⁰ m.

Il est choisi la résine la Dowex SD2, qui présente un spectre plus large d'élimination des molécules contaminantes (autres que les PGN) que d'autres résines de même famille.

Les solutions de polymères de glucose à 32 % (250 mL) sont éluées sur une colonne contenant 20 mL de cette résine.

La dernière étape consiste en une filtration sur membrane d'ultrafiltration présentant un seuil de coupure de 5 kDa.

Le traitement par ultrafiltration a pour objectif d'éliminer les molécules de petite taille encore présentes dans les solutions de polymères de glucose. La société Demanderesse recommande d'utiliser ce traitement en fin de procédé, car ce traitement a également un effet de dialyse qui permet d'éliminer les traces de sels accumulés au cours des traitements précédents.

Il est choisi d'injecter en continu la solution de polymères de glucose sur un filtre 5 kDa à un débit de 25 mL/min pendant 3 h à température ambiante. Pour compenser la perte du filtrat, le rétentat est injecté dans la solution de départ et ajusté en continu au volume initial (100 mL) par addition de tampon PBS stérile. Après 3 h, le volume de filtrat est compris entre 150 et 200 mL, ce qui est supérieur au volume initial de la solution de polymère de glucose.

Comme il sera exemplifié ci-après, cette combinaison d'étapes est seule apte à fournir une protection maximale des circuits de production de polymères et de glucose et leurs dérivés à l'encontre des contaminants d'origine bactérienne.

L'invention sera mieux comprise à l'aide des exemples qui suivent, lesquels se veulent illustratifs et non limitatifs.

### Exemples

### Exemple 1 : Etablissement des courbes doses-réponses

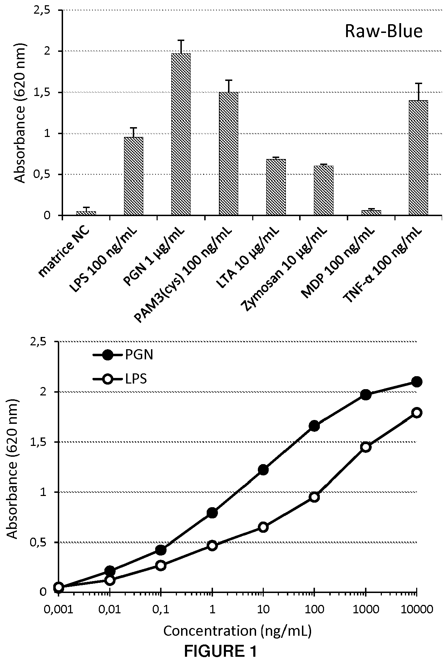

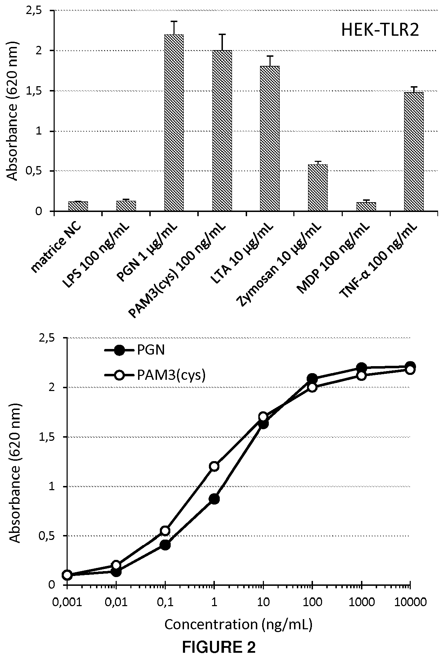

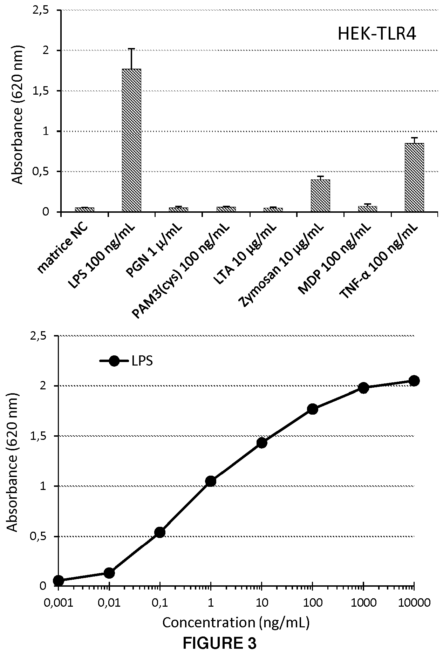

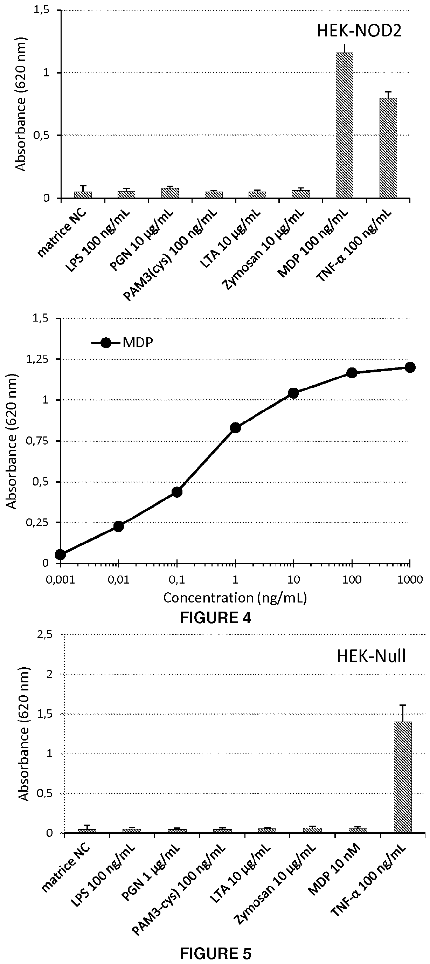

Les courbes doses-réponses sont réalisées avec des molécules agonistes standards : LPS, PGN, PAM3(cys) (pour PAM₃Cys-Ser-(Lys)4 trihydrochloride, un lipopeptide de synthèse), LTA, zymosan et MDP. Les lignées Raw-Blue^{™} et HEK-Blue^{™} hTLR2, hTLR4, hNOD2 et Null sont incubées avec des concentrations croissantes en agonistes, et la réponse cellulaire est mesurée par quantification de l'activité SEAP. Le TNF-α est utilisé comme témoin positif d'activation des cellules :
- Lignée Raw-Blue^{™} (Figure 1) : les cellules répondent aux molécules inflammatoires majeures susceptibles d'être présentes dans les matrices et dérivés de polymères de glucose (PGN, lipopeptides, LPS, zymosan, LTA) ; elles ont notamment une forte réactivité vis à vis des PGN, mais ne répondent pas à ses produits de dépolymérisation.
- Lignée HEK-Blue^{™} hTLR2 (Figure 2) : forte réactivité vis-à-vis des PGN et du lipopeptide PAM3(cys) ; les cellules répondent plus faiblement aux autres ligands de TLR2 (LTA, zymosan) et ne montrent aucune réactivité vis-à-vis des LPS et du MDP,
- Lignée HEK-Blue^{™} hTLR4 (Figure 3) : forte réactivité vis-à-vis des LPS ; les cellules répondent très faiblement au zymosan et ne montrent aucune réactivité vis-à-vis des PGN, lipopeptide, LTA et MDP,
- Lignée HEK-Blue^{™} hNOD2 (Figure 4) : forte réactivité vis-à-vis du MDP,
- Lignée HEK-Blue^{™} Null2 (Figure 5) : témoin d'absence de toxicité cellulaire.

### Exemple 2 : Préparation des différentes matrices de polymères de glucose

Comme indiqué ci-avant, les matrices sont les suivantes :
- 4 polymères de glucose, matières premières de l'Icodextrine (avant fractionnement chromatographique selon l'enseignement du brevet EP 667.356) référencés ici E1565, E3063, E1242 et E5248

La préparation de ces polymères est réalisée conformément aux enseignements de la demande de brevet WO 2012/059685.
- un lot d'icodextrine contaminé (référencé ici E209J) et un lot d'icodextrine "contrôle négatif", i.e. témoin de non-contamination dans les tests cellulaires (référencé ici P-11.11). Ces lots sont préparés selon l'enseignement du brevet EP 667.356, détaillé dans l'exemple 1 de la demande de brevet WO 2010/125315.

### Exemple 3 : Analyse des réponses cellulaires induites par les échantillons non traités

L'objectif de ces essais est de déterminer la réactivité pro-inflammatoire et la nature des contaminants présents dans les différentes matrices.

Les échantillons selon l'exemple 2 sont préparés à 32 % (poids/volume) dans de l'eau apyrogène (*p.p.i.*).

Les dosages des taux de LPS et de PGN ont été réalisés préalablement aux tests cellulaires en utilisant les dosages SLP-HS et LAL (données présentées ci-dessous) :

| | P11-11 | E1242 | E1565 | E3063 | E5248 | E209J |
|---|---|---|---|---|---|---|
| PGN (ng/g) SLP-HS | < 3 | 21 | 2320 | 16185 | 116 | 393 |
| LPS (EU/g) LAL | < 0,3 | 2,4 | 38,4 | 2,4 | 9,6 | 0,6 |
| LPS (EU/g) LAL modifié | < 0,3 | 1,2 | 4,8 | 1,2 | 0,3 | < 0,3 |

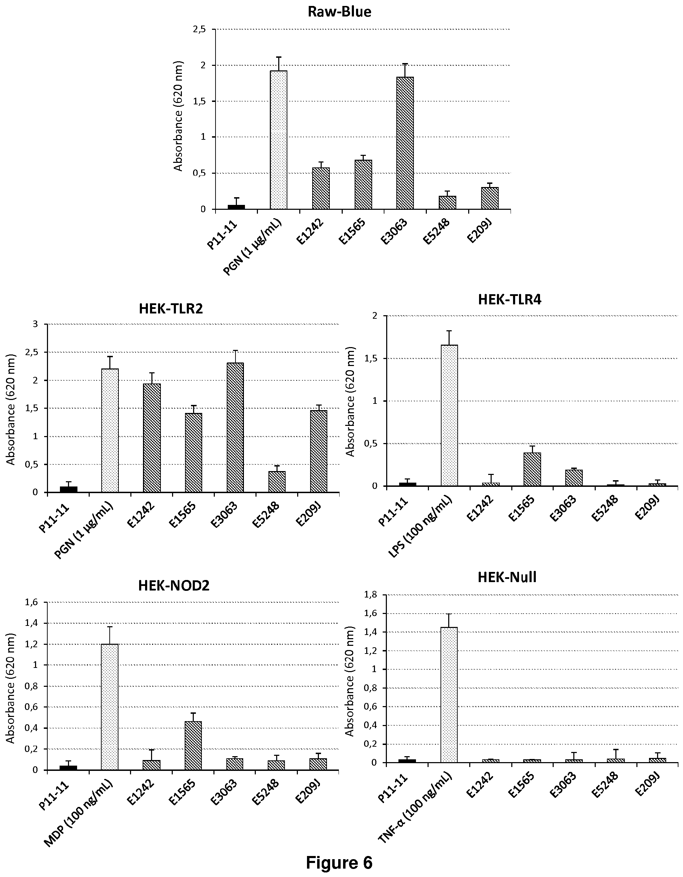

La présence de bio-contaminants dans les différentes matrices a été analysée à l'aide des cinq types cellulaires, de façon à avoir un aperçu des réponses inflammatoires à certains contaminants (Figure 6).

Pour les tests cellulaires, les échantillons sont dilués au 1/10 dans le milieu de culture des cellules (concentration finale : 3,2 % (p/v)).

Les analyses sont réalisées sur :
- Lignée Raw-Blue^{™} : tous contaminants avec réactivité élevée pour les PGN,
- Lignée HEK-Blue^{™}hTLR2 : réactivité élevée pour les PGN et les lipopeptides,
- Lignée HEK-Blue^{™}hTLR4 : réactivité élevée pour les LPS,
- Lignée HEK-Blue^{™}hNOD2 : MDP et produits de dépolymérisation des PGN,
- Lignée HEK-Blue^{™}Null2 : témoin d'absence de toxicité cellulaire.

### Bilan par échantillon :

- E1242 : réponse inflammatoire d'intensité élevée dans les cellules HEK-TLR2, et faible dans les cellules HEK-NOD2, témoin d'une forte contamination en PGN peu dégradé.
- E1565 : réponse inflammatoire d'intensité élevée dans les cellules HEK-TLR2, moyenne dans les cellules HEK-TLR4 et forte dans les cellules HEK-NOD2, témoin d'une forte contamination en PGN dégradé et en LPS.
- E3063 : réponse inflammatoire saturée dans les cellules HEK-TLR2, d'intensité moyenne dans les cellules HEK-TLR4 et d'intensité faible dans les cellules HEK-NOD2, témoin d'une très forte contamination en PGN peu dégradé et de traces de LPS.
- E5248 : réponse inflammatoire d'intensité faible dans les différentes lignées, témoin d'une faible contamination en PGN et en LPS.
- E209J : réponse inflammatoire d'intensité élevée dans les cellules HEK-TLR2 et faible dans les cellules HEK-NOD2, témoin d'une forte contamination en PGN faiblement dégradé.

Aucune matrice de polymères de glucose ne donne de réponse en présence des cellules HEK-Null, ce qui confirme l'absence de cytotoxicité.

### Exemple 4 : Analyse de l'effet des procédures de décontamination sur les réponses cellulaires induites par les échantillons

Dans sa demande de brevet internationale WO 2013/178931, la société Demanderesse a permis l'identification des traitements les mieux adaptés à chaque type de contaminants présents dans les échantillons et de déterminer les conditions expérimentales pour leur application sur des matrices de polymères de glucose.

Sur la base de cette étude antérieure, les nombreux traitements unitaires proposés sont :
- traitement sur charbons actifs,
- ultrafiltration sur membrane de seuil de filtration 5 kDa,
- passage sur résines d'adsorptions (,
- traitement par des préparations enzymatiques.

Dans les expériences de combinaisons décrites ci-après, le choix et l'agencement des étapes a été déterminé en fonction :
∘ des conditions expérimentales de traitement,
∘ des niveaux de contamination dans les échantillons et
∘ de la nature des molécules pro-inflammatoires.

### Utilisation de la Mannaway^{®}

Les enseignements de la demande de brevet internationale WO 2013/178931 ont montré que la préparation enzymatique est légèrement contaminée par des traces de LPS. En outre, des traces d'enzyme peuvent subsister après traitement dans la solution de polymères de glucose. De façon à éliminer ces contaminations exogènes, la société Demanderesse recommande donc de placer l'étape de traitement enzymatique en début de procédure dans les essais de combinaisons.

### Utilisation des charbons actifs

Les charbons actifs retenus pour la présente étude sont :
∘ C-extra-USP, pour son efficacité à éliminer les PGN et leurs produits de dégradation ;
∘ ENO-PC, pour son action préférentielle sur les contaminants de poids moléculaire < 100 kDa (par exemple, LPS et produits de dégradation des PGN) ;
∘ A-Supra-Eur, pour son efficacité à éliminer les complexes de poids moléculaire élevé.

La société Demanderesse a choisi d'utiliser les charbons actifs seuls ou associés par paire dans les différentes combinaisons et, étant donné que les traitements par charbons sont réalisés en *batch* et nécessitent des étapes de chauffage, neutralisation et filtration, ils sont réalisés juste après le traitement enzymatique, mais avant les autres traitements.

### Utilisation des résines

Les résines retenues sont :
∘ Macronet MN-150, pour son efficacité à retenir les molécules de bas poids moléculaire (par exemple, MDP et produits de dégradation des PGN) ;
∘ Dowex SD2, pour son spectre large d'élimination des contaminants, à l'exception des PGN.

### Utilisation de la séparation membranaire

Le traitement par ultrafiltration 5 kDa pour objectif d'éliminer les molécules de petite taille encore présentes dans les solutions de polymères de glucose. De plus, cette procédure a un effet de dialyse et permet d'éliminer les traces de sels accumulés au cours des traitements précédents.

Cette étape est donc placée systématiquement en fin de procédure.

Après chaque étape, des prélèvements sont réalisés en condition stérile et utilisés dans les tests cellulaires, afin de doser la charge inflammatoire globale (réponse des cellules Raw-Blue^{™}) et les quantités de bio-contaminants (réponses des HEK-Blue^{™}). Les réponses cellulaires obtenues après chaque étape sont comparées à la réponse induite par la matrice de départ, de façon à estimer l'efficacité des procédures de décontamination. Les résultats sont exprimés en activité par rapport à la réponse maximale des cellules. Dans tous les essais, un contrôle de non-contamination est réalisé avec une solution d'icodextrine P-11.11.

### Exemple 5 : Analyse comparative de différentes combinaisons des étapes unitaires de traitement

Un grand nombre de combinaisons possibles peuvent être implicitement déduites de l'enseignement de la demande de brevet WO 2013/178931.

Les résultats obtenus sur 3 des procédures que l'on pourrait concevoir sont les suivants.

### - Procédure 1 : matrice E1242 ; traitement par charbons actifs C-Extra USP + ENO-PC ; passage sur résine Macronet MN-15; ultrafiltration sur 5 kDa

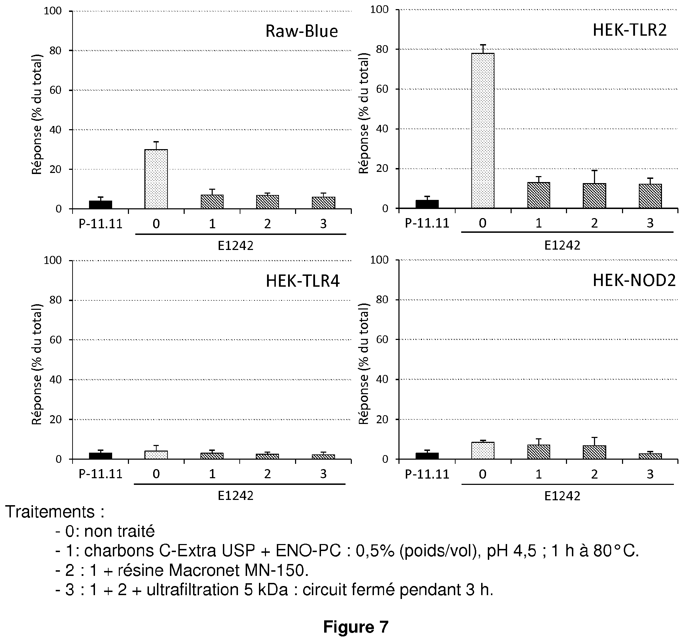

Les résultats des tests cellulaires sont présentés en Figure 7.

La matrice E1242 induit une réponse inflammatoire moyenne dans les cellules Raw-Blue, qui est majoritairement liée à une forte contamination en PGN (réponse TLR2).

Elle contient aussi des traces de LPS et de produits de dégradation des PGN, étant donné que les réponses TLR4 et NOD2 sont proches du bruit de fond.

La procédure de décontamination réduit efficacement les réponses inflammatoires.

Toutefois, la réponse des cellules Raw-Blue reste légèrement supérieure au contrôle de non-contamination (P-11.11), indiquant que des traces de contaminants sont encore présentes.

Ce résultat est dû à la présence de PGN résiduels après décontamination. En effet, la réponse TLR2 est fortement réduite après traitement par les charbons, mais reste supérieure au contrôle négatif.

Les autres étapes n'ont pas d'effet sur la réponse TLR2, qui n'évolue plus jusqu'à la fin de la procédure. Par contre, la réponse TLR4 n'est plus détectable dès la 1^{ère} étape de traitement, preuve que les LPS ont été éliminés. Quant à la réponse NOD2, elle est supprimée après l'étape d'ultrafiltration.

### Conclusion :

Bien que la charge en composés inflammatoires soit réduite de manière efficace, la procédure 1 n'est pas suffisante pour assurer une décontamination complète d'une matrice fortement chargée en PGN.

### - Procédure 2 : matrice E209J ; traitement par charbons actifs C-Extra USP + ENO-PC ; passage sur résine Dowex SD2 : ultrafiltration sur 5 kDa

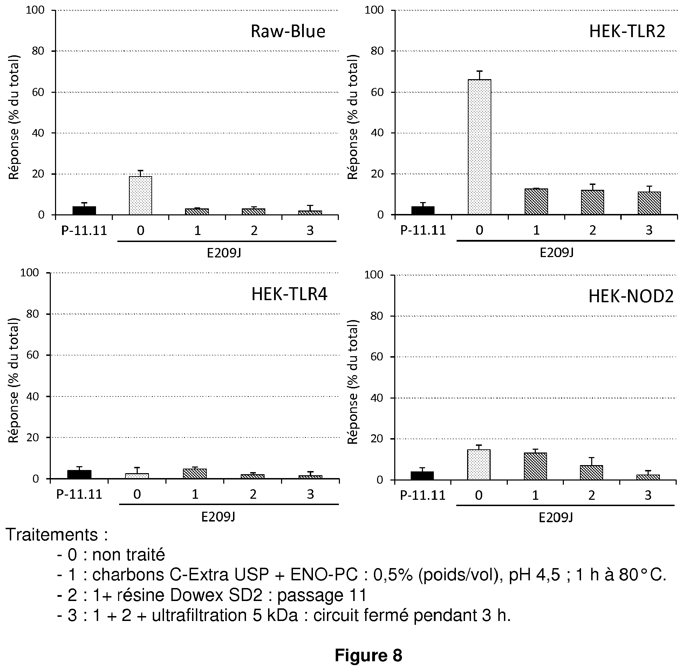

Les résultats des tests cellulaires sont présentés en Figure 8.

Pour cette procédure, la résine a été remplacée par la Dowex SD2, qui présente un spectre de rétention plus large. Les essais ont été réalisés avec la matrice E209J, qui présente un profil de contamination similaire à E1242.

Dans ce cas, la réponse des cellules Raw-Blue est identique au contrôle négatif, preuve que la décontamination a été efficace.

Toutefois, la réponse TLR2 est encore supérieure au contrôle de non-contamination, ce qui indique que des traces de PGN sont encore présentes.

La différence de réponses est certainement liée au fait que les cellules HEK-TLR2 ont un seuil de détection plus bas que les cellules Raws-Blue pour les PGN.

La réponse NOD2 est diminuée après passage sur SD2 et supprimée après ultrafiltration, ce qui suggère que cette résine a au moins une action complémentaire pour éliminer les produits de dégradations des PGN.

### Conclusion

Ces données indiquent que le changement de résine n'a pas amélioré l'efficacité de la procédure à éliminer tous les PGN.

### - Procédure 3 : matrice E5248 ; traitement par Mannaway^{®} puis par charbons C-Extra USP + A-Supra-Eur ; passage sur résine Dowex SD2 ; ultrafiltration sur 5 kDa

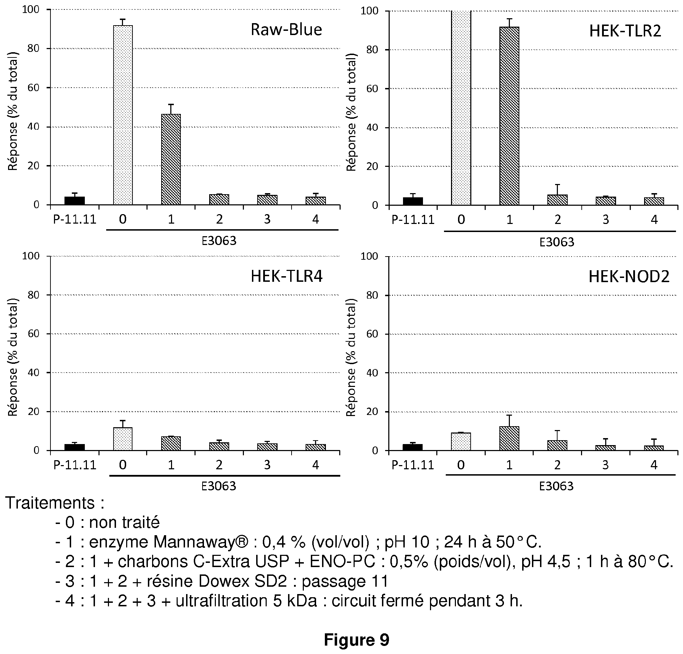

Les résultats des tests cellulaires sont présentés en Figure 9.

Dans cette dernière combinaison, le charbon A-Supra-Eur a été associé au C-Extra USP en remplacement du ENO-PC.

Contrairement à ce dernier, la charbon A-Supra-Eur élimine préférentiellement les molécules de poids moléculaire élevé.

Cette combinaison devrait être efficace pour décontaminer des échantillons chargés en macro-complexes agrégés de type PGN ou β-glucanes.

Les essais ont été réalisés sur la matrice E5248, qui induit une réponse inflammatoire d'intensité faible dans les différentes lignées, alors que les dosages SLP-HS et LAL suggèrent la présence d'une forte contamination en PGN et/ou en β-glucanes.

Cette différence peut s'expliquer par la masse élevée des molécules inflammatoires, ce qui diminuerait leur solubilité, et par conséquent leur accessibilité pour induire une réponse dans les tests cellulaires.

Après la 1^{ère} étape de la procédure, une forte augmentation des réponses cellulaires est observée dans les différentes lignées.

Ce résultat confirme que le traitement enzymatique par la Mannaway^{®} est efficace pour désagréger des complexes ou débris bactériens, libérant ainsi des molécules agonistes de TLR2, TLR4 et NOD2.

Après traitement aux charbons, les réponses des cellules Raws et HEK-TLR2 sont nettement diminuées mais restent au-dessus du contrôle négatif.

De même, les réponses TLR4 et NOD2 restent conséquentes, et il faut attendre le passage sur la résine et l'étape finale d'ultrafiltration pour obtenir un signal identique au contrôle de non-contamination dans les différents types cellulaires.

### Conclusion

Ces résultats indiquent que le charbon A-Supra-Eur n'a pas apporté de bénéfice notable dans la procédure de décontamination.

### Conclusion finale

Bien que tout à fait concevables, aucune de ces procédures de décontamination ne donne de résultat complètement satisfaisant.

### Exemple 6 : Présentation de la procédure optimisée

Il est choisi de réaliser sur la matrice E3063 la succession des étapes suivantes :
- traitement par Mannaway^{®} puis
- par charbons actifs C-Extra USP + ENO-PC, puis
- passage sur résine Dowex SD2 et enfin
- ultrafiltration sur 5 kDa

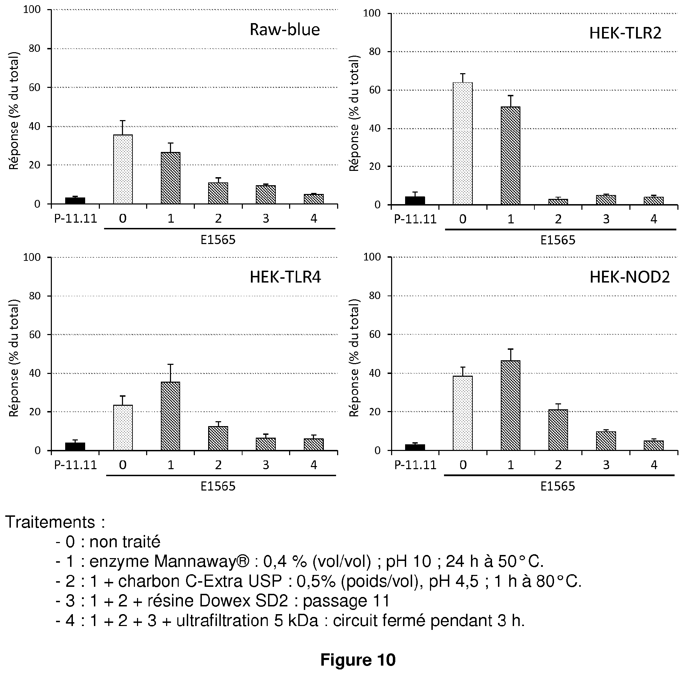

Les résultats des tests cellulaires sont présentés en Figure 10.

Pour augmenter l'efficacité de la procédure visant à éliminer les PGN, une étape de traitement enzymatique par la Mannaway^{®} est introduite en amont des autres étapes.

Pour ces essais, la matrice E3063, qui est très fortement chargée en PGN, a été utilisée.

Après les différentes étapes de la procédure, la réponse des cellules Raw-Blue est identique au contrôle négatif, preuve que la décontamination a été efficace.

En outre, l'association des traitements enzyme + charbons est particulièrement adaptée à l'élimination des PGN, puisque la réponse TLR2 passe d'un signal saturé avant traitement à un signal identique au contrôle de non-contamination.

Finalement, les autres étapes sont efficaces pour éliminer les traces de LPS (réponse TLR4) et d'agonistes de NOD2.

Afin de démontrer que cette combinaison est bien efficace, le charbon ENO-PC a été retiré de façon à vérifier si son utilisation apporte réellement un bénéfice à la procédure de décontamination.

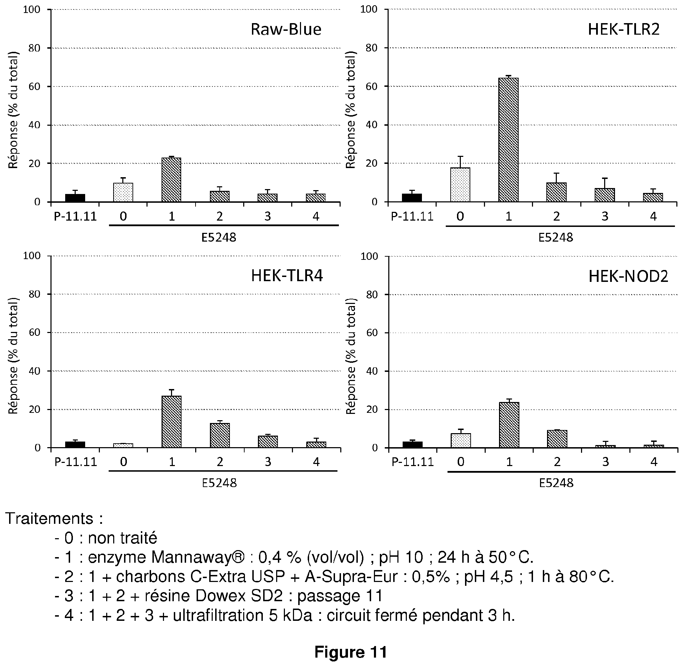

Les résultats des tests cellulaires sont présentés en Figure 11.

Les essais ont été réalisés sur une autre matrice, la matrice E1565, qui est contaminée par les différents agonistes de TLR2, TLR4 et NOD2.

En fin de procédure, la réponse des cellules Raw-Blue est nettement diminuée, mais reste légèrement supérieure au contrôle de non-contamination. Cette réponse très faible n'est pas liée à la présence de PGN résiduel, mais plutôt à des traces de LPS et d'agonistes de NOD2, et à un possible effet synergique entre les deux familles de molécules.

Le charbon ENO-PC possède un spectre large de rétention pour des molécules de poids moléculaire < 100 kDa (LPS et produits de dégradation des PGN).

### Conclusion

On constate que son absence a réduit l'efficacité de la procédure de décontamination, notamment si la matrice est fortement contaminée par ces deux familles de molécules inflammatoires.

Dans leur ensemble, les résultats obtenus dans cette étude montrent que la combinaison de plusieurs étapes de décontamination judicieusement sélectionnées et ordonnées se révèle efficace pour éliminer les molécules inflammatoires susceptibles d'être présentes dans des solutions de polymères de glucose.

La combinaison comprend les étapes suivantes :
- traitement par une préparation enzymatique à propriétés détergentes et de clarification, par exemple Mannaway^{®} ,
- traitement par un charbon actif de porosité équivalente à C-Extra-USP,
- facultativement, traitement par un second charbon actif de porosité équivalente à ENO-PC pour les matrices chargées en LPS et/ou PGN dégradés,
- passage sur une résine d'adsorption de type Dowex-SD2,
- ultrafiltration en continu sur 5 kDa.

Les étapes sélectionnées permettent de cibler les différentes familles de contaminants et de proposer des produits exempts de réactivité inflammatoire.

### Figures

**Figure 1** : Réponses des cellules Raw-Blue^{™} aux agonistes standards.
**Figure 2** **:** Réponses des cellules HEK-Blue^{™}TLR2 aux agonistes standards.
**Figure 3** **:** Réponses des cellules HEK-Blue^{™}TLR4 aux agonistes standards.
**Figure 4** **:** Réponses des cellules HEK-Blue^{™}NOD2 aux agonistes standards.
**Figure 5** **:** Réponses des cellules HEK-Blue^{™}Null aux agonistes standards.
**Figure 6** : Réponses cellulaires induites par les matrices de polymères de glucose.
**Figure 7** Réponses cellulaires induites par la matrice E1242 après décontamination selon la procédure 1.
**Figure 8** Réponses cellulaires induites par la matrice E209J après décontamination selon la procédure 2.
**Figure 9** Réponses cellulaires induites par la matrice E5248 après décontamination selon la procédure 5
**Figure 10** **:** Réponses cellulaires induites par la matrice E3063 après décontamination selon la procédure 3.
**Figure 11** Réponses cellulaires induites par la matrice E1565 après décontamination selon la procédure 4.

## Revendications

1. Procédé de décontamination des polymères de glucose ou leurs hydrolysats de leurs molécules pro-inflammatoires, **caractérisé en ce qu'**il comprend les étapes suivantes
a) fournir des polymères de glucose ou leurs hydrolysats ;
b) facultativement, détecter ou doser les molécules pro-inflammatoires dans les polymères de glucose ou leurs hydrolysats fournis à l'étape a) ;
c) effectuer les étapes de purification suivantes dans l'ordre suivant:
i. traitement par une préparation enzymatique à activité mannanase,
ii. traitement par un charbon actif de très haute capacité d'adsorption, de qualité pharmaceutique, de porosité « micropore »,
iii. facultativement, traitement par un second charbon actif de porosité « mésopore »,
iv. passage sur une résine adsorbante polymérique macroporeuse, présentant une porosité supérieure à 100 × 10⁻¹⁰ m, et
v. ultrafiltration en continu sur 5 kDa,
dans lequel les polymères de glucose sont sélectionnés parmi l'icodextrine et les maltodextrines.

2. Procédé selon la revendication 1, **caractérisé en ce que** le procédé comprend les 5 étapes i à v.

3. Procédé selon la revendication 1 ou 2, **caractérisé en ce que** les maltodextrines sont des maltodextrines branchées ou non, et les hydrolysats de polymères de glucose sont un produit d'hydrolyse totale tel le dextrose monohydrate.

## Patentansprüche

1. Verfahren zur Dekontaminierung von Glukosepolymeren oder deren Hydrolysaten von ihren entzündungsfördernden Molekülen, **dadurch gekennzeichnet, dass** es die folgenden Schritte umfasst:
a) Bereitstellen von Glukosepolymeren oder deren Hydrolysaten;
b) gegebenenfalls Nachweisen oder Messen der entzündungsfördernden Moleküle in den in Schritt a) bereitgestellten Glukosepolymeren oder deren Hydrolysaten;
c) Durchführen der folgenden Reinigungsschritte in folgender Reihenfolge:
i. Behandlung mit einem Enzympräparat mit Mannaseaktivität,
ii. Behandlung mit einer Aktivkohle mit sehr hoher Adsorptionskapazität, pharmazeutischer Qualität, mit "mikroporöser" Porosität,
iii. gegebenenfalls Behandlung mit einer zweiten Aktivkohle mit "mesoporöser" Porosität,
iv. Passage über ein makroporöses polymeres Adsorberharz mit einer Porosität von mehr als 100 × 10⁻¹⁰ m, und
v. kontinuierliche Ultrafiltration über 5 kDa,
wobei die Glucosepolymere aus Icodextrin und Maltodextrinen ausgewählt sind.

2. Verfahren nach Anspruch 1, **dadurch gekennzeichnet, dass** das Verfahren die fünf Schritte i bis v umfasst.

3. Verfahren nach Anspruch 1 oder 2, **dadurch gekennzeichnet, dass** die Maltodextrine verzweigte oder unverzweigte Maltodextrine sind und die Hydrolysate von Glucosepolymeren ein vollständiges Hydrolyseprodukt wie Dextrose-Monohydrat sind.

## Claims

1. A method for decontaminating glucose polymers or hydrolysates thereof of the pro-inflammatory molecules thereof, **characterised in that** it comprises the following steps:
a) providing glucose polymers or hydrolysates thereof;
b) optionally, detecting or assaying the pro-inflammatory molecules in the glucose polymers or the hydrolysates thereof provided in step a);
c) carrying out the following purification steps in the following order:
i. treatment by an enzyme preparation with mannanase activity,
ii. treatment by a pharmaceutical-grade activated carbon with a very high adsorption capacity and a "microporous" porosity,
iii. optionally, treatment by a second activated carbon with a "mesoporous" porosity,
iv. passing over a macroporous adsorbent polymer resin having a porosity of greater than 100 × 10⁻¹⁰ m, and
v. continuous 5 kDa ultrafiltration,
wherein the glucose polymers are selected from icodextrin and maltodextrins.

2. The method according to claim 1, **characterised in that** the method comprises the 5 steps i to v.

3. The method according to claim 1 or 2, **characterised in that** the maltodextrins are branched or unbranched maltodextrins, and the glucose polymer hydrolysates are a product of total hydrolysis such as dextrose monohydrate.
